# EUROPÄISCHE PATENTSCHRIFT

(11) **EP 0 523 418 B1**
(45) Veröffentlichungstag und Bekanntmachung des Hinweises auf die Patenterteilung: **02.10.1996**
(21) Anmeldenummer: 92110705.8
(22) Anmeldetag: 25.06.1992
(51) Int. Cl.: A61K 9/127, A61K 7/00

(54) **Verfahren zur Herstellung von stabilen, hydrophilen oder ambiphilen Creme-Zubereitungen, enthaltend vesikuläre Bestandteile, und deren Verwendung**
Process for preparing stable hydrophilic or ambiphic cream preparations containing vesicular composnents and their use
Procédé pour la fabrication de crèmes stables hydrophiles ou ambiphiles contenant des composants vesiculaires et leur utilisation

(30) Priorität: 03.07.1991 DE 4121945
(43) Veröffentlichungstag der Anmeldung: 20.01.1993
(73) Patentinhaber: Merz & Co. GmbH & Co., D-60318 Frankfurt (DE)
(72) Erfinder: Nürnberg, Eberhard, Prof. Dr., W-8525 Uttenreuth-Weiher (DE); Paspaleeva-Kühn, Valentina, W-8520 Erlangen (DE); Beutler, Rolf D., Dr., W-6128 Höchst/Odw. (DE); Wimmer, Thomas, Dr., W-6000 Frankfurt am Main 90 (DE)
(74) Vertreter: Wolff, Hans Joachim, Dr.jur. Dipl.-Chem.

(56) Entgegenhaltungen:
- EP-A- 0 140 085
- EP-A- 0 424 282
- WO-A-88/06883
- FR-A- 2 624 374
- GB-A- 2 143 433
- SÖFW: SEIFEN, ÖLE, FETTE, WACHSE Bd. 116, Nr. 2, 1. Februar 1990, AUGSBURG (DE) Seiten 51 - 55 , XP136052 A. BRUNKE 'spezifische eigenschaften von sphingosomen'

## Beschreibung

Die vorliegende Erfindung betrifft das in den Patentansprüchen beschriebene Verfahren zur Herstellung von stabilen, hydrophilen oder ambiphilen Creme-Zubereitungen, welche vesikuläre Bestandteile wie ionische oder nichtionische Vesikel und/oder Liposomen enthalten und "klassische" Öl-in-Wasser-Cremes darstellen. Die Erfindung betrifft ferner die Verwendung der Creme-Zubereitungen für die kosmetische oder dermatologische Anwendung.

Üblicherweise werden Vesikel- und/oder Liposomen-enthaltende kosmetische bzw. pharmazeutische Zubereitungen auf Hydrogel-Basis hergestellt, die geringfügige Mengen an lipophilen Bestandteilen enthalten können. In solchen Gel-Systemen sind Vesikel und/oder Liposomen stabil zu halten.

Die DE-PS 35 37 723 beschreibt Zusammensetzungen für die kosmetische oder pharmazeutische Anwendung, welche nichtionische Partikel (Niosome) als zwingenden Bestandteil und mindestens ein wasserlösliches Polyamid enthalten. Die Zusammensetzungen stellen 2 bis 10%ige Niosomen- bzw. Niosomen-Liposomen-Dispersionen dar. Hierbei werden in der äußeren wäßrigen Phase der Vesikel-Dispersion vorzugsweise 20-40% Öl, bezogen auf das Gesamtgewicht der Zusammensetzung, dispergiert. Halbfeste Systeme können erreicht werden, wenn anstelle flüssiger Öle Vaseline verwendet wird oder Polyacrylzusätze eingearbeitet werden.

Bei diesen Formulierungen handelt es sich jedoch nicht um "klassische" Cremes, da außer den Vesikel-bildenden Bestandteilen keine weiteren Tenside enthalten sind.

In "klassischen" Öl-in-Wasser-Cremes liegt eine Stabilisierung der Fettphase und der halbfesten Konsistenz durch ein lamellares, kohärentes Netzwerk aus Öl-in-Wasser- und Wasser-in-Öl-Tensiden vor, wie von K.H.Bauer, K.H.Frömming und C. Führer in Pharmazeutische Technologie, 1989, S. 324 und von J. Juninger, C.Führer, J.Ziegenmeyer und S.Friberg in J.Soc.Cosmet.Chem., 30 (1979), S. 9 beschrieben. Werden in solche Systeme Liposomen oder Vesikel eingearbeitet, so werden diese durch Wechselwirkung mit den wasserlöslichen Tensiden solubilisiert und lösen sich auf (vgl. H. Lautenschläger, J.Röding, M.Ghyczy, Seifen, Öle, Fette, Wachse, 114 (1988), S. 531-534 sowie A.Helenius, K.Simons, Biochem.Biophys.Acta, 415 (1975), S. 59). Stabile klassische Öl-in-Wasser-Cremes, enthaltend vesikuläre Bestandteile, sind auf diese Weise nicht erhältlich.

Die FR-A 2 624 374 und die EP-A 0 424 282 betreffen Sonnenschutzpräparate, wobei der aktive Wirkstoff in Liposomen inkorporiert ist. Cremezubereitungen werden hierbei auf übliche Weise hergestellt durch Einsatz einer Kombination aus W/O-Emulgatoren (HLB-Wert kleiner 9, Lanolin und Cetyl/Stearylalkohol), sowie Polyoxyethylenstearat, welches wasserlöslich sein muß, um mit den W/O-Produkten eine stabile Emulsion zu ergeben und somit einen HLB-Wert größer 13 aufweisen muß. Diese sind jedoch, wie erwähnt, der Liposomenstabilität abträglich.

Aufgabe vorliegender Erfindung ist es daher, ein einfaches und wirtschaftliches Verfahren zur Herstellung stabiler, hydrophiler oder ambiphiler Cremes bereitzustellen, welche vesikuläre Bestandteile enthalten.

Diese Aufgabe wird erfindungsgemäß dadurch gelöst, daß man der Fettphase der Zubereitung ein nichtionisches, nicht klar wasserlösliches, jedoch wasserdispergierbares polyoxyethyliertes Tensid, welches einen HLB-Wert von 9-13 aufweist, zusetzt. Die so bereitete Fettphase wird anschließend mit der Wasserphase, die die darin löslichen Bestandteile enthält, in üblicher Weise emulgiert. Anschließend wird der Mischung der gewünschte Gehalt an vesikulären Bestandteilen, insbesondere an ionischen und nichtionischen Vesikeln und/oder Liposomen, zugesetzt.

Überraschenderweise zeigte sich, daß bei Zusatz des Tensids, vorzugsweise in einer Menge von 5 bis 20%, insbesondere 5-10 %, bezogen auf die Gesamtmenge der Zubereitung, stabile "klassische" Öl-in-Wasser-Cremes erhalten werden, aus denen die vesikulären Bestandteile nicht herausgelöst werden.

Insbesondere bevorzugt wird die unter der Bezeichnung "Macrogol-stearat 400" bekannte Verbindung als Tensid eingesetzt.

Geeignet sind zudem auch andere polyoxyethylierte Ether und Ester sowie Silikontenside, wenn bei einem HLB-Wert von 9-13 eine Wasserdispergierbarkeit gegeben ist.

Der Zusatz an Tensid mit den obengenannten Eigenschaften bewirkt, daß die Fettphase von lamellaren Schichten des Tensids, die ein dreidimensionales Netzwerk aufbauen, umgeben ist, was Transmissions-Elektronenmikroskopische Aufnahmen zeigen. Dabei resultieren überraschenderweise Strukturen, die sonst nur durch die übliche oben beschriebene Kombination von Öl-in-Wasser- und Wasser-in-Öl-Emulgatoren zu erzielen sind.

Die so hergestellten Creme-Zubereitungen sind noch nach mehreren Monaten Lagerung stabil, die intakten Vesikel sind im Netzwerk (Transmissions-Elektronenmikroskopisch) nachweisbar.

Als Fettphasenbestandteile eignen sich übliche, für die Creme-Zubereitung bekannte lipophile Substanzen. Vorzugsweise werden Isopropylpalmitat, Perhydrosqualen, Paraffin, Isopropylmyristat, Cetylpalmitat, Oleyloleat, mittelkettige Triglyceride, Octyldodecanol und pflanzliche Öle in Mengen von 5-20%, insbesondere 5-18%, bezogen auf die Gesamtmenge der Zubereitung, eingesetzt.

Die Fettphasenbetandteile und das Tensid werden bei geeigneter Temperatur, im Falle fester nichtionischer Tenside bei dessen Schmelztemperatur, gemischt. Bevorzugt erfolgt dies bei 60°C bis 90°C, inbesondere bei 70°C bis 75°C. Hierbei werden in der Salben- und Creme-Herstellung übliche Herstellungsapparaturen verwendet.

Anschließend wird die wäßrige Phase, welche übliche wasserlösliche Bestandteile wie Propylenglykol, Polyethylenglykol, Sorbitol oder Glycerol enthält, auf die gleiche Temperatur wie die Fettphase erhitzt, dieser unter Rühren zugegeben und in üblicher Weise emulgiert.

Der wäßrigen Phase bzw. der Fettphase können je nach Hydro- bzw. Lipophilie weitere Zusätze wie Kolloide, Konservierungsstoffe, Antioxidantien und/oder Parfümöle zugesetzt werden. Als Kolloide eignen sich insbesondere Collagene, Elastin, Collagenhydrolysate, Gelatine, Hyaluronsäure oder Polyacrylsäuren wie z.B. Carbomer 934, 940, 941 sowie 980, die in Mengen von 1-8%, insbesondere 4-6%, bezogen auf die Gesamtmenge der Zubereitung, eingesetzt werden können.

Geeignete Konservierungsmittel sind beispielsweise Phenoxethol, Sorbinsäure, insbesondere Kaliumsorbat, oder Parabene, die in üblichen Mengen zugesetzt werden. Als Antioxidans kann beispielsweise Vitamin E oder Butylhydroxytoluol in üblichen Mengen zugesetzt werden.

Daneben können auch kosmetische und/oder pharmazeutische Wirkstoffe enthalten sein, die jedoch auch zusammen mit den Liposomen und/oder Vesikeln eingebracht werden können. Solche Wirkstoffe sind beispielsweise fettlösliche Vitamine wie ₐ-Tocopherol bzw. dessen Acetat, Vitamin-A oder dessen Palmitat, ferner Corticosteroide, Nucleinsäure oder andere Substanzen vom DNA- bzw. RNA-Typ, Aknetherapeutika wie Tretinoin, oder andere topisch applizierbare Wirkstoffe wie Clotrimazol und ähnliches. Die Wirkstoffe können üblicherweise in Mengen von 0,1 bis 10 g, vorzugsweise 0,2 bis 2 g pro 100 g Gesamtmenge der Zubereitung eingesetzt werden.

Nach der Herstellung der Öl-in-Wasser-Emulsion werden die vesikulären Bestandteile, wahlweise die obengenannten Wirkstoffe enthaltend, bei geeigneten Temperaturen unter Rühren hinzugegeben. Vorzugsweise erfolgt dies bei 20°C bis 50°C, wobei übliche Apparaturen verwendet werden können.

Als Liposomen bzw. Vesikel eignen sich die aus der Herstellung diese enthaltender Gele bekannten ionischen oder nichtionischen Bestandteile, wie z.B. Ei- oder Sojalecithin, oder synthetisch hergestellte oder native C₁₆-C₂₀-Phosphatidylcholine, wobei der Fettsäureanteil gesättigt oder ungesättigt sein kann.

Diese Vesikel und/oder Liposome können nach literaturbekannten Verfahren, wie z.B. von D.E.Billek in Seifen, Öle, Fette Wachse, 113 (1987), S. 469-473 zusammengestellt, hergestellt werden. Gegebenenfalls kann eine Ultraschallbehandlung vorgenommen werden.

Die Homogenisierung der Bestandteile kann in einem Hochdruckhomogenisator vorgenommen werden. 10%ige und 20%ige Liposomendispersionen sind zudem im Handel erhältlich.

Gegebenenfalls können im erfindungsgemäßen Verfahren vesikuläre Partikel, die nach dem Verfahren gemäß der DE 41 11 982.7 hergestellt werden, eingesetzt werden. Die üblichen Einsatzkonzentrationen der vesikulären Partikel liegen bei 5-20%, insbesondere 5-10%, einer 10%igen Dispersion, bezogen auf die Gesamtmenge der Zubereitung, vorhanden. Ganz besonders bevorzugt sind 10 bzw. 20%ige wäßrige Liposomendispersionen, die in einer Menge von 5%, bezogen auf die Gesamtmenge der Zubereitung, eingearbeitet werden.

Die gegebenenfalls mit pharmazeutischen oder kosmetischen Wirkstoffen versehene, wie oben geschildert hergestellte stabile Creme-Zubereitung kann als dermatologisches oder kosmetisches Mittel verwendet werden.

Da die vesikulären Bestandteile in einer Creme-Formulierung enthalten sind, können diese effektiver als mit bisherigen einfachen Gel-Zubereitungen angewendet werden. Dabei können erfindungsgemäß hydrophile Öl-in-Wasser-Emulsionen mit dem Tensidzusatz aber auch ambiphile Cremes mit einer kohärenten Wasserphase zubereitet werden, ohne daß ein Herauslösen der Vesikel erfolgt. Somit kann je nach Bedarf eine entsprechend auf die Anforderung abgestimmte optimale Formulierung zubereitet werden.

Die Erfindung wird anhand der nachfolgenden Beispiele näher erläutert.

### Beispiel 1

### Herstellung einer vesikelfreien Emulsion

Die nachfolgend genannten Bestandteile werden in den angegebenen Mengen wie folgt miteinander umgesetzt:

| | |
|---|---|
| Macrogol-stearat 400 | 5,0 |
| Isopropylpalmitat | 5,0 |
| Perhydrosqualen | 12,0 |
| Tocopherolacetat | 0,2 |
| Propylenglykol | 4,0 |
| Collagen-Lösung | 4,0 |
| Parfümöl | 0,2 |
| Kaliumsorbat | 0,1 |
| Sorbinsäure | 0,1 |
| Wasser | ad 100 |

Zunächst wird das nichtionische Tensid Macrogol-stearat 400 mit den Fettbestandteilen Isopropylpalmitat und Perhydrosqualen bei 70°C bis 75°C geschmolzen. Die auf 70°C bis 75°C erhitzte Wasserphase wird mit Propylenglykol und Konservans unter Rühren der Fettphase zugegeben und emulgiert.

Hierbei werden übliche Salben- oder Creme-Herstellungsapparaturen verwendet.

Nach dem Abkühlen erhält man eine "klassische" Öl-in-Wasser-Emulsion.

### Beispiel 2

Nach dem Verfahren gemäß Beispiel 1 wurde eine Öl-in-Wasser-Creme aus den nachstehend genannten Bestandteilen hergestellt:

| | |
|---|---|
| Macrogol-stearat 400 | 5,0 |
| Isopropylpalmitat | 5,0 |
| Perhydrosqualen | 12,0 |
| Tocopherolacetat | 0,2 |
| Propylenglykol | 4,0 |
| Collagen-Lösung | 4,0 |
| Parfümöl | 0,2 |
| Kaliumsorbat | 0,1 |
| Sorbinsäure | 0,1 |
| Wasser | ad 95,0 |

Anschließend wurde eine handelsübliche 10%ige Liposomen-Dispersion aus Sojalecithin bei einer Temperatur von 30°C bis 35°C in üblicher Weise in die Creme-Formulierung eingearbeitet, wobei ein Gehalt von 5% an Liposomen, bezogen auf die Gesamtmenge, erhalten wird.

Man erhält eine lagerstabile, hydrophile Öl-in-Wasser-Creme mit Vitaminzusatz und Parfümöl für die topische Anwendung auf der Haut.

### Beispiel 3

Nach der Verfahrenweise gemäß Beispiel 2 wurde eine Liposomen-haltige, wirkstoffhaltige Creme-Zubereitung der folgenden Zusammensetzung hergestellt:

| | |
|---|---|
| Macrogol-stearat 400 | 5,0 |
| Oleyloleat | 5,0 |
| Paraffin, dickflüssig | 12,0 |
| Tocopherolacetat | 0,2 |
| Vitamin A-palmitat | 0,2 |
| Polyacrylsäure | 0,2 |
| Natriumhydroxid-Lösung, 5%ig | 1,2 |
| Propylenglykol | 4,0 |
| Collagen-Lösung | 5,0 |
| Liposomen-Dispersion, 10%ig | 5,0 |
| Parfümöl | 0,2 |
| Kaliumsorbat | 0,1 |
| Sorbinsäure | 0,1 |
| Wasser | ad 100 |

Die handelsübliche 10%ige Liposomendispersion wurde bei 35°C bis 45°C mit der Creme-Formulierung zusammengemischt.

### Beispiel 4

Nach der Verfahrensweise gemäß Beispiel 3 wurde eine Creme der folgenden Zusammensetzung zubereitet:

| | |
|---|---|
| Macrogol-stearat 400 | 5,0 |
| Isopropylpalmitat | 5,0 |
| Perhydrosqualen | 12,0 |
| Tocopherolacetat | 0,2 |
| Natriumhydroxid-Losung, 5%ig | 3,0 |
| Propylenglykol | 4,0 |
| Liposomen-Dispersion, 20%ig | 5,0 |
| Parfümöl | 0,2 |
| Kaliumsorbat | 0,1 |
| Sorbinsäure | 0,1 |
| Wasser | ad 100 |

Die handelsübliche 20%ige Liposomendispersion wurde mit der Öl-in-Wasser-Emulsion bei 20°C bis 30°C vermengt.

Es werden lagerstabile Formulierungen erhalten, worin die intakten Vesikel noch nach mehreren Monaten im nichtionischen Netzwerk nachweisbar sind.

## Patentansprüche

1. Verfahren zur Herstellung von stabilen, hydrophilen oder ambiphilen Creme-Zubereitungen, enthaltend vesikuläre Bestandteile, dadurch gekennzeichnet, daß man der Fettphase der Creme-Zubereitung ein nichtionisches, nicht klar wasserlösliches, wasserdispergierbares polyoxyethyliertes Tensid mit einem HLB-Wert von 9 bis 13 zusetzt, diese Phase mit der Wasserphase emulgiert und die vesikulären Bestandteile unter geeigneten Bedingungen und gegebenenfalls übliche weitere Zusatzstoffe und/oder Wirkstoffe hinzufügt.

2. Verfahren gemäß Anspruch 1, dadurch gekennzeichnet, daß als nichtionisches Tensid Macrogol-stearat 400 verwendet wird.

3. Verfahren gemäß Anspruch 1 oder 2, dadurch gekennzeichnet, daß das nichtionische polyoxyethylierte Tensid in einer Menge von 5 bis 20 %, vorzugsweise 5 bis 10%, bezogen auf die Gesamtmenge der Creme-Zubereitung, verwendet wird.

4. Verfahren gemäß einem der Ansprüche 1 bis 3, dadurch gekennzeichnet, daß man der Creme-Zubereitung weitere Zusatzstoffe hinzufügt.

5. Verfahren gemäß Anspruch 4, dadurch gekennzeichnet, daß man als Zusätze Kolloide, Konservierungsmittel, Antioxidantien und/oder kosmetische oder pharmazeutische Wirkstoffe einsetzt.

6. Verfahren gemäß Anspruch 5, dadurch gekennzeichnet, daß man als Kolloide Collagene, Collagenhydrolysate, Gelatine oder Polyacrylsäuren, als Konservierungsmittel Sorbinsäure oder Phenoxethol, als Antioxidans Vitamin E und als Wirkstoff ein oder mehrere fettlösliche Vitamine einsetzt.

7. Verfahren gemäß einem der Ansprüche 1 bis 6, dadurch gekennzeichnet, daß die Herstellung der Fettphase bei der Schmelztemperatur des nichtionischen polyoxyethylierten Tensids erfolgt und die Einarbeitung der vesikulären Bestandteile bei 20°C bis 50°C erfolgt.

8. Creme-Zubereitung, umfassend eine Fettphase, enthaltend ein polyoxyethyliertes Tensid, eine Wasserphase sowie vesikuläre Bestandteile und gegebenenfalls übliche Zusatzstoffe und/oder Wirkstoffe, erhältlich nach dem Verfahren gemäß einem der Ansprüche 1-7.

9. Verwendung einer Creme-Zubereitung, hergestellt nach dem Verfahren gemäß einem der Ansprüche 1 bis 7, zur Herstellung eines Mittels für die kosmetische oder dermatologische Anwendung.

## Claims

1. A process for producing stable, hydrophilic or ambiphilic cream preparations containing vesicular constituents, characterised in that a nonionic, water-dispersible, polyoxyethylated surfactant, which is not readily water-soluble and which has an HLB value from 9 to 13, is added to the fat phase of the cream preparation, the latter phase is emulsified with the aqueous phase and the vesicular constituents are added under suitable conditions, and customary further additives and/or active ingredients are optionally added.

2. A process according to claim 1, characterised in that macrogol stearate 400 is used as a nonionic surfactant.

3. A process according to claims 1 or 2, characterised in that the nonionic polyoxyethylated surfactant is used in an amount of 5 to 20 %, preferably 5 to 10 %, with respect to the total amount of the cream preparation.

4. A process according to any one of claims 1 to 3, characterised in that further additive materials are added to the cream preparation.

5. A process according to claim 4, characterised in that colloids, preservatives, antioxidants and/or cosmetic or pharmaceutical active ingredients are used as additives.

6. A process according to claim 5, characterised in that collagens, collagen hydrolysates, gelatins or polyacrylic acids are used as colloids, sorbic acid or phenoxethol are used as preservatives, vitamin E is used as an antioxidant, and one or more fat-soluble vitamins are used as an active ingredient.

7. A process according to any one of claims 1 to 6, characterised in that production of the fat phase is effected at the melting temperature of the nonionic polyoxyethylated surfactant and incorporation of the vesicular constituents is effected at 20°C to 50°C.

8. A cream preparation comprising a fat phase, containing a polyoxyethylated surfactant, an aqueous phase and vesicular constituents, and optionally customary additives and/or active ingredients, obtainable by the process according to any one of claims 1 to 7.

9. Use of a cream preparation produced by the process according to any one of claims 1 to 7 for producing a medium for cosmetic or dermatological application.

## Revendications

1. Procédé pour la fabrication de crèmes stables hydrophiles ou ambiphiles contenant des composants vésiculaires, caractérisé en ce que l'on ajoute à la phase grasse de la crème un agent tensioactif polyoxyéthylique non-ionique, peu soluble dans l'eau, dispersable dans l'eau, ayant une valeur HLB de 9 à 13, on émulsionne cette phase avec la phase aqueuse et on ajoute les composants vésiculaires sous des conditions adéquates et, le cas échéant, d'autres additifs et/ou matières actives habituelles.

2. Procédé selon la revendication 1, caractérisé en ce que l'on utilise comme agent tensioactif non-ionique du Macrogol-stearat 400

3. Procédé selon la revendication 1 ou 2, caractérise en ce que l'agent tensioactif non-ionique polyoxyéthylique est utilisé à raison de 5 à 20 %, de préférence 5 à 10 %, par rapport à la masse totale de la crème.

4. Procédé selon l'une des revendications 1 à 3, caractérisé en ce que l'on ajoute d'autres additifs à la crème.

5. Procédé selon la revendication 4, caractérisé en ce que l'on utilise comme additifs des colloïdes, des agents conservateurs, des agents anti-oxydants et/ou des matières actives cosmétiques ou pharmaceutiques.

6. Procédé selon la revendication 5, caractérisé en ce que l'on utilise comme colloïdes des collagènes, des hydrolysats de collagène, de la gélatine ou des acides polyacryliques, comme agents conservateurs de l'acide sorbique ou du phénoxétol, comme agents antioxydants de la vitamine E et, comme matière active, une ou plusieurs vitamines liposolubles.

7. Procédé selon l'une des revendications 1 à 6, caractérisé en ce que la fabrication de la phase grasse s'effectue à la température de fusion de l'agent tensioactif non-ionique polyoxyéthylique et l'incorporation des composants vésiculaires à une température comprise entre 20°C et 50°C.

8. Crème comprenant une phase grasse contenant un agent tensioactif polyoxyéthylique, une phase aqueuse ainsi que des composants vésiculaires et, le cas échéant, des additifs et/ou matières actives habituelles, que l'on peut obtenir selon le procédé conforme à l'une des revendications 1 à 7.

9. Utilisation d'une crème fabriquée selon le procédé conforme à l'une des revendications 1 à 7, pour la fabrication d'un agent destiné à l'application cosmétique ou dermatologique.
